(19) Europäisches Patentamt
European Patent Office
Office européen des brevets

(11) EP 0 461 574 B1

(12) **EUROPÄISCHE PATENTSCHRIFT**

(45) Veröffentlichungstag und Bekanntmachung des Hinweises auf die Patenterteilung:
**24.09.1997 Patentblatt 1997/39**

(21) Anmeldenummer: **91109449.8**

(22) Anmeldetag: **08.06.1991**

(51) Int Cl.[6]: **C07D 471/08**, C07D 471/10, A61K 31/435
// (C07D471/08, 221:00, 221:00), (C07D471/10, 221:00, 221:00)

(54) **3,7-Diazabicyclo(3,3,1)-nonan-Verbindungen enthaltende Arzneimittel**

Medicines containing 3,7-diazabicyclo(3,3,1)nonanes

Médicaments contenant des 3,7-diazabicyclo(3,3,1)nonanes

(84) Benannte Vertragsstaaten:
**AT BE CH DE DK ES FR GB GR IT LI LU NL SE**

(30) Priorität: **15.06.1990 DE 4019080**

(43) Veröffentlichungstag der Anmeldung:
**18.12.1991 Patentblatt 1991/51**

(73) Patentinhaber: **Solvay Pharmaceuticals GmbH
30173 Hannover (DE)**

(72) Erfinder:
- **Burow, Kurt
W-3000 Hannover 61 (DE)**
- **Buschmann, Gerd
W-3000 Hannover 72 (DE)**
- **Farjam, Arman
W-3101 Wienhausen (DE)**
- **Kühl, Ulrich
W-3007 Gehrden 1 (DE)**
- **Varchmin, Gerda
W-3000 Hannover 1 (DE)**
- **Ziegler, Dieter
W-3003 Ronnenberg 1 (DE)**
- **Schön, Uwe
W-3167 Burgdorf (DE)**

(74) Vertreter: **Lauer, Dieter, Dr.
Solvay Pharmaceuticals GmbH,
Hans-Böckler-Allee 20
30173 Hannover (DE)**

(56) Entgegenhaltungen:
**EP-A- 0 000 074** **EP-A- 0 306 871**
**FR-A- 2 375 235**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 461 574 B1

Printed by Jouve, 75001 PARIS (FR)

## Beschreibung

Die vorliegende Erfindung betrifft die Verwendung von 3,7,9,9-tetrasubstituierten 3,7-Diazabicyclo[3,3,1]nonan-Verbindungen zur Herstellung von diuretisch wirksamen Arzneimitteln, sowie neue 3,7,9,9-tetrasubstituierte 3,7-Diazabicyclo[3,3,1]nonan-Verbindungen mit wertvollen pharmakologischen Eigenschaften, insbesondere diuretischen Wirkungen.

Aus EP-A-0 103 833 sind durch aliphatische Reste 3,7,9,9-tetrasubstituierte 3,7-Diazabicyclo[3,3,1]nonan-Verbindungen mit herzwirksamen, insbesondere antiarrhythmischen Eigenschaften bekannt. EP-A-0 306 871 beschreibt 3,7,9,9-tetrasubstituierte 3,7-Diazabicyclo[3,3,1]nonan-Verbindungen, welche in 3-Stellung eine gegebenenfalls substituierte Benzhydryl- oder Cinnamylgruppe tragen und herzwirksame, insbesondere bradycarde und calciumantagonistische Eigenschaften besitzen und einen günstigen Einfluß auf den Herzrhythmus haben. In EP-A-0 301 245 werden 3,7,9,9-tetrasubstituierte 3,7-Diazabicyclo[3,3,1]nonan-Verbindungen beschrieben, welche in 3-Stellung eine Benzylgruppe tragen und als Zwischenprodukte zur Herstellung von in 3-Stellung einen substituierten Sulfonylrest tragenden 3,7,9,9-tetrasubstituierten 3,7-Diazabicyclo[3,3,1]nonan-Verbindungen mit die Magenmotilität beeinflussenden pharmakologischen Wirkungen dienen. Aus EP-A-0 000 074 sind in 9-Stellung unsubstituierte 3,7-Diazabicyclo[3,3,1]nonan-Verbindungen mit antiarrhythmischen Eigenschaften bekannt. Aus FR-A-2 375 235 sind 7-Acyl-3,7-diazabicyclo [3,3,1]nonan-Verbindungen mit das zentrale Nervensystem beeinflussenden, insbesondere analgetischen Eigenschaften bekannt.

Der Erfindung liegt die Aufgabe zugrunde, neue diuretisch wirksame pharmazeutische Zubereitungen mit verbessertem Wirkungsprofil zu entwickeln.

Erfindungsgemäß werden zur Herstellung von pharmakologischen Zubereitungen zur diureseverstärkenden Behandlung von Krankheitszuständen, bei denen eine vermehrte Harnausscheidung und eine erhöhte Salzausscheidung zur Ausschwemmung von Ödemen erforderlich sind, als diuretische Wirkstoffe 3,7-Diazabicyclo[3,3,1]nonan-Verbindungen der allgemeinen Formel I

(s. Formel I). worin

- $R^1$ eine Alkylgruppe mit 1-6 Kohlenstoffatomen, eine Alkenylgruppe mit 3-6 Kohlenztoffatomen deren Doppelbindung nicht direkt an das Stickstoffatom gebunden ist, eine Cycloalkylalkylgruppe mit 4-9 Kohlenstoffatomen oder Benzyl bedeutet,
- $R^2$ $C_1$ - $C_4$- Alkyl bedeutet und
- $R^3$ $C_1$ - $C_4$- Alkyl bedeutet oder
- $R^2$ und $R^3$ gemeinsam eine Alkylenkette mit 3-6 Kohlenstoffatomen bilden, und
- $R^4$ eine Alkylgruppe mit 1-6 Kohlenstoffatomen, eine Alkenylgruppe mit 3-6 Kohlenstoffatomen, deren Doppelbindung nicht direkt an das Stickstoffatom gebunden ist, oder eine Cycloalkylalkylgruppe mit 4-9 Kohlenstoffatomen bedeutet, oder
- $R^4$ eine Gruppe der allgemeinen Formel a

(s. Formel a)

darstellt, worin

- $R^5$ Wasserstoff, Halogen, $C_1$ - $C_4$- Alkyl oder $C_1$ - $C_4$-Alkoxy bedeutet und
- Z eine Alkylenkette mit 1-3 Kohlenstoffatomen oder die Propenylenkette, deren Doppelbindung zum Phenylrest konjugiert ist, bedeutet, oder
- $R^4$ eine Gruppe der allgemeinen Formel b

(s. Formel b)

darstellt, worin

- $R^6$ Wasserstoff, Halogen, $C_1$ - $C_4$- Alkyl oder $C_1$ - $C_4$ - Alkoxy und
- $R^7$ Wasserstoff, Halogen, $C_1$ - $C_4$- Alkyl oder $C_1$ - $C_4$- Alkoxy bedeuten,

und deren physiologisch verträgliche Säureadditionssalze verwendet.

Sofern in den Verbindungen der Formel I $R^1$ für eine $C_{1-6}$Alkylgruppe steht, kann diese geradkettig oder verzweigt sein und vorzugsweise 3-6 Kohlenstoffatome enthalten. Eine $C_{3-6}$ Alkenylgruppe $R^1$ kann ebenfalls geradkettig oder verzweigt sein und vorzugsweise 3 oder 4 Kohlenstoffatome enthalten. Eine $C_{4-9}$ Cycloalkylalkylgruppe $R^1$ kann vorzugsweise 4-7 Kohlenstoffatome enthalten und besteht insbesondere aus einer durch einen Cycloalkylrest mit 3-6 Kohlenstoffatomen substituierten Alkylenkette mit 1-3 Kohlenstoffatomen, vorzugsweise einer cycloalkylsubstituierten Methylengruppe. Als besonders geeignete Reste $R^1$ haben sich Alkylreste, insbesondere verzweigte Alkylreste, und

Cycloalkylmethylreste erwiesen.

Sofern die Substituenten $R^2$ und $R^3$ $C_{1-4}$ Alkyl darstellen, können diese Alkylgruppen geradkettig oder verzweigt sein und vorzugsweise 1-3 Kohlenstoffatome enthalten. Die Alkylgruppen $R^2$ und $R^3$ sind zweckmäßigerweise gleichartig. Sofern $R^2$ und $R^3$ gemeinsam eine $C_{3-6}$ Alkylenkette bilden, kann diese vorzugsweise 4-5 Kohlenstoffatome enthalten.

Sofern in den Verbindungen der Formel I der Rest $R^4$ für eine $C_{1-6}$ Alkylgruppe steht, kann diese geradkettig oder verzweigt sein und vorzugsweise 3-6 Kohlenstoffatome enthalten. Eine $C_{3-6}$ Alkenylgruppe $R^4$ kann geradkettig oder verzweigt sein und vorzugsweise 3 oder 4 Kohlenstoffatome enthalten. Eine $C_{4-9}$ Cycloalkylalkylgruppe $R^4$ kann vorzugsweise 4-7, Kohlenstoffatome enthalten und stellt insbesondere eine durch Cycloalkyl mit 3-6 Kohlenstoffatomen substituierte Alkylenkette mit 1-3 Kohlenstoffatomen, vorzugsweise eine cycloalkylsubstituierte Methylengruppe dar. Als besonders geeignete Reste $R^4$ haben sich Alkylreste, insbesondere verzweigte Alkylreste, und Cycloalkylmethylreste erwiesen.

In den Verbindungen der Formel I kann der Rest $R^4$ auch eine gegebenenfalls substituierte Phenylalkyl- oder Cinnamylgruppe der Formel a darstellen. Sofern der Substituent $R^5$ der Phenylgruppe $C_{1-4}$ Alkyl darstellt oder enthält, kann dieses insbesondere 1 oder 2 Kohlenstoffatome enthalten. Ein Halogensubstituent $R^5$ stellt vorzugsweise Fluor oder Chlor dar. Die Kette Z mit 1-3 Kohlenstoffatome ist insbesondere eine Methylengruppe oder eine Propenylenkette, deren Doppelbindung zum Phenylrest konjugiert ist, darstellen.

In den Verbindungen der Formel I kann der Rest $R^4$ auch eine gegebenenfalls substituierte Benzhydrylgruppe b darstellen. Sofern die Substituenten $R^6$ und/oder $R^7$ der Benzhydrylgruppe b $C_{1-4}$ Alkylgruppen darstellen oder enthalten, können diese insbesondere 1 oder 2 Kohlenstoffatome enthalten. Halogensubstituenten stellen vorzugsweise Fluor oder Chlor dar.

Als physiologisch verträgliche Säureadditionssalze der Verbindungen der Formel I eignen sich Salze mit anorganischen Säuren, z. B. Halogenwasserstoffsäuren, insbesondere Chlorwasserstoffsäure, oder Schwefelsäure, oder mit organischen Säuren, beispielsweise niederen aliphatischen Mono- oder Dicarbonsäuren wie Essigsäure, Fumarsäure, Weinsäure, Milchsäure, Maleinsäure oder Zitronensäure, oder Salicylsäure, oder Sulfonsäuren, beispielsweise Niederalkylsulfonsäuren wie Methansulfonsäure oder gegebenenfalls im Benzolring durch Halogen oder niederes Alkyl substituierten Benzolsulfonsäuren, wie p-Toluolsulfonsäure.

Gegenstand der Erfindung sind die Verwendung der Verbindungen der Formel I als diuretisch wirksame pharmakologische Wirkstoffe zur Herstellung von Arzneimitteln zur diureseverstärkenden Behandlung.

3,7-Diazabicyclo[3,3,1]nonan-Verbindungen der allgemeinen Formel Ia

(s. Formel Ia) worin $R^1$, $R^2$ und $R^3$ obige Bedeutung besitzen und $R^{4'}$ eine Gruppe der allgemeinen Formel a'

(s. Formel a')

darstellt, worin

n 1-3 bedeutet und

$R^{5'}$ Halogen, $C_{1-4}$ Alkyl, $C_{1-4}$ Alkoxy oder, falls n 2 oder 3 ist, auch Wasserstoff bedeutet,

sind bisher in der Literatur noch nicht beschrieben worden und stellen neue wertvolle pharmakologische Wirkstoffe dar, welche ebenfalls Gegenstand der vorliegenden Erfindung sind.

Die übrigen erfindungsgemäß als diuretische Wirkstoffe verwendeten Verbindungen der Formel I fallen unter die Umfänge der in den vorgenannten europäischen Patentanmeldungen EP-A-0 103 883, EP-A-0 301 245 und EP-A-0 306 871 beschriebenen Verbindungen und sind aus diesen bekannt.

Es wurde nun überraschenderweise gefunden, daß die 3,7,9,9-tetrasubstituierten 3,7-Diazabicyclo[3,3,1]nonan-Verbindungen der Formel I sich durch ein neuartiges Wirkungsprofil auszeichnen und neben den vorgenannten herzwirksamen Eigenschaften auch eine ausgeprägte diuretische Wirkung besitzen. Dabei zeichnen sich die erfindungsgemäß verwendeten Verbindungen ferner durch eine gute Verträglichkeit und ein günstiges Verhältnis zwischen Natrium- und Kaliumausscheidung aus. Die diuretische Wirkung der Verbindungen der Formel I läßt sich in pharmakologischen Tests in vivo an Tieren, beispielsweise Ratten, nachweisen.

Beschreibung der pharmakologischen Versuchsmethoden.

1. Diuresebestimmung an wachen Ratten

Die durch die Testsubstanzen induzierte Zunahme der Diurese wird an wachen Ratten bestimmt. Gruppen von je drei Tieren werden die Testsubstanzen in wäßriger Lösung oder Suspension per os verabreicht. Zusätzlich werden den Tieren der Testgruppen und Tieren einer Kontrollgruppe noch eine weitere Menge von jeweils 25 ml pro kg Körpergewicht Wasser per os verabreicht. Danach wird der Harn über 6 Stunden gesammelt. Die im Harn ausgeschiedene Natriummenge wird bestimmt und in Milliäquivalent pro 100 g Körpersubstanz berechnet. Als Faktor der durch die

Testsubstanz verursachten Diuresezunahme wird das Verhältnis von der durch die Testtiergruppen ausgeschiedenen Menge zu der durch die Kontrolltiergruppe ausgeschiedenen Menge angegeben.

Die mit einer repräsentativen Gruppe von Verbindungen der Formel I nach der vorstehend beschriebenen Methode erhaltenen Versuchsergebnisse werden in der nachstehenden Tabelle I wiedergegeben.

## Tabelle I

Substanzen der Formel I
Substituenten

| Nr. | $R^1$ | $R^2$ | $R^3$ | $R^4$ | Salz | Diuretische Wirkung: Dosis mg/kg p.o. | Diuretische Wirkung: Verstärkung der Diurese um Faktor |
|---|---|---|---|---|---|---|---|
| 1 | $n-C_6H_{13}-$ | $CH_3-$ | $CH_3-$ | $n-C_6H_{13}-$ | a | 20 | 1,8 |
| 2 | $n-C_3H_7-$ | $C_2H_5-$ | $C_2H_5-$ | $n-C_3H_7$ | a | 20 | 8,2 |
| 3 | $(CH_3)_2-CH-$ | $n-C_3H_7-$ | $n-C_3H_7-$ | $(CH_3)_2-CH-$ | a | 20 | 18,8 |
| 4 | cycloprop-$CH_2-$ | $-(CH_2)_4-$ | | cycloprop-$CH_2-$ | a | 20 | 9,8 |
| 5 | $n-C_4H_9-$ | $CH_3-$ | $C_2H_5-$ | $n-C_4H_9-$ | a | 20 | 4,9 |
| 6 | cyclohex-$CH_2-$ | $CH_3-$ | $C_2H_5-$ | cyclohex-$CH_2-$ | a | 20 | 8,8 |
| 7 | $n-C_4H_9-$ | $CH_3-$ | $CH_3-$ | $(CH_3)_2-CH-CH_2-$ | a | 20 | 5,0 |
| 8 | $n-C_4H_9-$ | $-(CH_2)_3-$ | | $n-C_4H_9$ | a | 20 | 3,7 |
| 9 | $CH_2=CH-CH_2-CH_2-$ | $-(CH_2)_5-$ | | $CH_2=CH-CH_2-CH_2-$ | c | 20 | 10,4 |
| 10 | $CH_2=CH-CH_2-CH_2-$ | $CH_3-$ | $CH_3-$ | $CH_2=CH-CH_2-CH_2-$ | c | 20 | 4,7 |
| 11 | $(CH_3)_2-CH-CH_2-$ | $n-C_3H_7-$ | $n-C_3H_7-$ | $CH_2=CH-CH_2-CH_2-$ | c | 20 | 6,3 |
| 12 | $n-C_4H_9-$ | $CH_3-$ | $CH_3-$ | $CH_2=CH-CH_2-CH_2-$ | c | 20 | 9,0 |
| 13 | $(CH_3)_2-CH-$ | $-(CH_2)_5-$ | | $(CH_3)_2-CH-CH_2-$ | a | 20 | 2,1 |
| 14 | phen-$CH_2-$ | $CH_3-$ | $CH_3-$ | phen-$CH_2-$ | d | 10 | 4,3 |
| 15 | $n-C_4H_9-$ | $CH_3-$ | $CH_3-$ | phen-$CH_2-$ | c | 2,5 | 3,0 |
| 16 | $n-C_4H_9-$ | $CH_3-$ | $CH_3-$ | 3-Cl-phen-$CH_2-$ | c | 5 | 2,2 |
| 17 | $n-C_4H_9-$ | $CH_3-$ | $CH_3-$ | 2-Cl-phen-$CH_2-$ | c | 5 | 3,7 |
| 18 | $n-C_4H_9-$ | $CH_3-$ | $CH_3-$ | 4-$CH_3O$-phen-$CH_2-$ | c | 5 | 5,6 |
| 19 | $n-C_4H_9-$ | $CH_3-$ | $CH_3-$ | 2,5-di-$CH_3$-phen-$CH_2-$ | c | 10 | 2,6 |
| 20 | $n-C_4H_9-$ | $CH_3-$ | $CH_3-$ | 4-$CH_3$-phen-$CH_2-$ | c | 10 | 2,7 |
| 21 | phen-$CH_2-$ | $-(CH_2)_4-$ | | phen-$CH_2-$ | e | 20 | 2,8 |
| 22 | cycloprop-$CH_2-$ | $-(CH_2)_4-$ | | phen-$CH_2-$ | a | 20 | 2,4 |

cycloprop = Cyclopropyl,
phen = Phenyl,
cyclohex = Cyclohexyl,
diphe = Diphenylmethyl

a = Dihydrogentartrat,
b = Monohydrogentartrat,
c = Monosalicylat,
d = Monohydrogenfumarat,
e = Dihydrochlorid

**Tabelle I** (Fortsetzg.)

| Nr. | Substanzen der Formel I Substituenten R¹ | R² | R³ | R⁴ | Salz | Diuretische Wirkung Dosis mg/kg p.o. | Verstärkung der Diurese um Faktor |
|---|---|---|---|---|---|---|---|
| 23 | cyclohex-$CH_2$- | -$(CH_2)_4$- | | phen-$CH_2$- | a | 20 | 2,4 |
| 24 | $(CH_3)_2$-$CH_2$- | $CH_3$- | $CH_3$- | phen-$CH_2$- | a | 20 | 2,4 |
| 25 | n-$C_4H_9$- | $CH_3$- | $CH_3$- | phen-CH=CH-$CH_2$- | b | 10 | 3,8 |
| 26 | $(CH_3)_2$-CH-$CH_2$- | $CH_3$- | $CH_3$- | phen-CH=CH-$CH_2$- | a | 20 | 4,0 |
| | | | | | | 10 | 2,2 |
| 27 | cycloprop-$CH_2$- | $CH_3$- | $CH_3$- | phen-CH=CH-$CH_2$- | a | 20 | 2,2 |
| 28 | $(CH_3)_2$-CH-$CH_2$- | -$(CH_2)_4$- | | phen-CH=CH-$CH_2$- | a | 20 | 2,2 |
| 29 | $(CH_3)_2$-CH-$CH_2$- | $CH_3$- | $CH_3$- | 4-F,4'-F-diphe- | a | 1 | 2,1 |
| 30 | n-$C_4H_9$- | $CH_3$- | $CH_3$- | 4-F,4'-F-diphe- | a | 20 | 4,6 |
| | | | | | | 2,5 | 2,7 |
| 31 | $(CH_3)_2$-CH- | -$(CH_2)_4$- | | diphe- | a | 20 | 3,9 |
| | | | | | a | 2,5 | 2,2 |
| 32 | $(CH_3)_2$-CH- | -$(CH_2)_4$- | | 4-F,4'-F-diphe- | a | 20 | 3,4 |
| | | | | | | 5 | 2,2 |
| 33 | cyclohex-$CH_2$- | $CH_3$- | $CH_3$- | diphe- | a | 5 | 2,6 |
| 34 | $(CH_3)_2$-CH-$CH_2$- | -$(CH_2)_4$- | | diphe- | a | 5 | 2,5 |
| 35 | cycloprop-$CH_2$- | -$(CH_2)_4$- | | diphe- | a | 20 | 6,2 |
| | | | | | | 2,5 | 2,8 |
| 36 | phen-$CH_2$- | -$(CH_2)_4$- | | diphe- | a | 20 | 4,7 |
| | | | | | | 10 | 4,2 |
| 37 | phen-$CH_2$- | -$(CH_2)_4$- | | 4-F,4'-F-diphe- | a | 20 | 5,7 |
| | | | | | | 5 | 3,1 |
| 38 | n-$C_4H_9$- | $CH_3$- | $CH_3$- | diphe- | a | 2,5 | 3,0 |
| 39 | cycloprop-$CH_2$- | $CH_3$- | $CH_3$- | diphe- | b | 2,5 | 3,6 |
| 40 | cycloprop-$CH_2$- | $CH_3$- | $CH_3$- | 4-F,4'-F-diphe- | a | 20 | 5,9 |
| | | | | | | 2,5 | 2,1 |

2. Bestimmung der diuretischen, natriuretischen und kaliuretischen Wirkungen an narkotisierten Ratten.

Die Versuche werden an männlichen Wistar-Ratten mit einem Körpergewicht von 320-370 g ausgeführt. Die Tiere werden etwa 17 Stunden vor Versuchsbeginn nüchtern gesetzt, haben aber ständig freien Zugang zum normalen Leitungswasser.

Alle Tiere werden durch eine intraperitoneale Injektion von Urethan (1,3 g/kg) narkotisiert und anschließend tracheotomiert. Zur intravenösen Injektion von Testsubstanzen und zur Infusion von physiologischer Kochsalzlösung (6 ml/h) wird in die linke Vena femoralis ein Plastikschlauch eingebunden. Zur Registrierung des arteriellen Blutdrucks wird die linke Arteria femoralis kanüliert. Die Harnblase wird durch einen abdominalen Schnitt freigelegt und katheterisiert. Der Katheter wird so in der Harnblase fixiert, daß der größte Teil der Harnblase funktionell ausgeschaltet ist. Anschließend wird die Harnröhre unterbunden. Während der Präparation und für die Dauer der Versuche liegen die Tiere auf einem heizbaren Operationstisch. Die Körpertemperatur wird konstant bei 37 °C gehalten. In einer 5-minütigen Vorlaufphase wird der Harn gesammelt und es werden die darin ausgeschiedenen Mengen an Kalium und Natrium bestimmt. Anschließend werden die Testsubstanzen i.v. appliziert. Der unter Einwirkung der Testsubstanzen ausgeschiedene Harn wird während 12 Minuten gesammelt und die darin ausgeschiedenen Mengen an Natrium und Kalium werden bestimmt. Die nachfolgende Tabelle II gibt die nach der vorstehend beschriebenen Testmethode bei einer Dosis von 10 µmol/kg erhaltenen Werte für die Testsubstanzen Nr. 4 und 13 wieder.

Tabelle II

| | Testsubstanz Nr. | |
|---|---|---|
| Diuretische Wirkung | 4[e)] | 13[f)] |
| Harnausscheidung in mg/100 g Körpergewicht/min Kontrollwert der Vorphase | 1,22 | 1,09 |
| Testwert | 22,72 | 8,07 |
| $Na^+$ -Ausscheidung in µmol/100 g Körpergewicht/min Kontrollwert der Vorphase | 0,07 | 0,07 |
| Testwert | 3,47 | 1,53 |
| $K^+$ -Ausscheidung in µmol/100 g Körpergewicht/min Kontrollwert der Vorphase | 0,11 | 0,09 |
| Testwert | 1,02 | 0,64 |

e) = Dihydrochlorid,

f) = Dihydrogenfumarat

Aus den vorstehenden pharmakologischen Testergebnissen zeigt sich, daß die Verbindungen der Formel I eine ausgeprägte diuretische Wirkung besitzen. Aus Tabelle II geht hervor, daß bei der durch die Verbindungen bewirkte Diurese die Natrium-Ausscheidung um ein Vielfaches stärker zunimmt als die Kalium-Ausscheidung. Dies deutet auf eine diuretische Wirkung der Verbindungen mit physiologisch günstigem Natrium/Kalium-Ausscheidungsprofil hin. Auf Grund ihrer guten diuretischen Eigenschaften eignen sich die Substanzen als Diuretika in der Therapie von Krankheitszuständen, bei denen eine vermehrte Harnausscheidung und eine erhöhte Salzausscheidung zur Ausschwemmung von Oedemen erforderlich ist.

Die günstige Kombination von diuretischer Wirksamkeit mit herzwirksamen Eigenschaften der Substanzen der Formel I macht die Substanzen besonders geeignet zur Diureseverstärkung und Oedemausschwemmung im Rahmen einer Behandlung von Herzinsuffizienzen und Hypertonien. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich zur Applikation am Menschen und an größeren Säugetieren jedoch Arzneiformen mit einem Wirkstoffgehalt von 0.5 bis 50, insbesondere 1 bis 20 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Kapseln, Pulver, Granulate oder Dragees genannt oder auch Suppositorien. Feste Präparate können pharmazeutisch übliche anorganische und/oder organische Trägerstoffe, wie z.B. Talkum, Milchzucker oder Stärke neben pharmazeutisch üblichen Hilfsmitteln, beispielsweise Gleitmitteln oder Tablettensprengmitteln enthalten. Flüssige Präparate wie Lösungen, Suspensionen oder Emulsionen können die üblichen Verdünnungsmittel wie Wasser, Öle und/oder Suspensionsmittel wie Polyäthylenglycole und dergleichen enthalten. Es können zusätzlich weitere Hilfsstoffe zugegeben werden wie z.B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs-

und/oder Trägerstoffen in üblicher Weise gemischt und naß oder trocken granuliert werden. Je nach Art der verwendeten Zusatzstoffe kann gegebenenfalls auch durch einfaches Mischen ein tablettierbares Pulver erhalten werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die Verbindungen der Formel I können auf an sich bekannte Weise, beispielsweise nach den in den vorstehend zitierten europäischen Patentanmeldungen beschriebenen Verfahren oder analog zu diesen Verfahren, hergestellt werden.

Beispielsweise können Verbindungen der Formel I erhalten werden, indem man

a) Verbindungen der allgemeinen Formel II
(s. Formel II)
worin $R^1$, $R^2$ und $R^3$ obige Bedeutung besitzen, mit Verbindungen der allgemeinen Formel III
(s. Formel III)
worin $R^4$ obige Bedeutung besitzt und X eine aminolytisch abspaltbare Gruppe bedeutet, umsetzt, oder

b) zur Herstellung von Verbindungen der allgemeinen Formel Ib
(s. Formel Ib)
worin $R^1$, $R^2$ und $R^3$ obige Bedeutung besitzen, und $R^{4}$ " eine Alkylgruppe mit 1-6 Kohlenstoffatomen, eine Cycloalkylalkylgruppe mit 4-9 Kohlenstoffatomen oder eine Gruppe der allgemeinen Formel a"
(s. Formel a")
bedeutet, worin n und $R^5$ obige Bedeutung besitzen, Verbindungen der allgemeinen Formel IV
(s. Formel IV)
worin $R^1$, $R^2$, $R^3$ und $R^{4}$ " obige Bedeutung besitzen, reduziert,
und gewünschtenfalls freie Verbindungen der Formel I in ihre physiologisch verträglichen Säureadditionssalze überführt oder die Säureadditionssalze in die freien Verbindungen der Formel I überführt.

Die Umsetzung der Verbindungen der Formel II mit Verbindungen der Formel III kann auf an sich bekannte Weise unter zur Alkylierung von Aminen üblichen Bedingungen erfolgen. So wird die Umsetzung zweckmäßigerweise in einem unter den Reaktionsbedingungen inerten organischen Lösungsmittel unter basischen Bedingungen durchgeführt. Als aminolytisch abspaltbare Reste in den Verbindungen der Formel III kommen bevorzugt Halogene wie Chlor oder Brom oder auch organische Sulfonsäurereste in Frage, beispielsweise Reste von Niederalkansulfonsäuren wie z.B. Methansulfonsäure oder von aromatischen Sulfonsäuren wie Benzolsulfonsäuren oder durch niederes Alkyl oder Halogen substituierten Benzolsulfonsäuren, z.B. Toluolsulfonsäuren oder Brombenzolsulfonsäuren. Als inerte organische Lösungsmittel eignen sich insbesondere aprotische Lösungsmittel wie beispielsweise Äther, insbesondere cyclische Äther wie Tetrahydrofuran, Dimethylformamid oder aromatische Kohlenwasserstoffe. Zweckmäßigerweise wird die Reaktion in Gegenwart mindestens einer äquivalenten Menge einer Base durchgeführt. Beispiele geeigneter Basen sind Alkalimetallcarbonate, Alkalimetallamide, Alkalimetallhydride oder lithiumorganische Verbindungen wie Niederalkyllithium oder Phenyllithium. So erweist sich beispielsweise die Verwendung von Lithiumamid in Tetrahydrofuran oder Dimethylformamid als zweckmäßig. Die Reaktionstemperatur kann je nach Art der verwendeten Base variieren und zwischen 0 °C und Siedetemperatur des Lösungsmittels gewählt werden.

Die Reduktion der Tetraoxoverbindungen der Formel IV kann nach an sich bekannten Methoden, beispielsweise unter Verwendung von komplexen Metallhydriden als Reduktionsmittel erfolgen. Als Reduktionsmittel gut geeignet ist z.B. Lithiumaluminiumhydrid. Vorteilhaft wird die Reduktion in einem unter dem Reaktionsbedingungen inerten organischen Lösungsmittel, beispielsweise einem cyclischen Äther wie Tetrahydrofuran oder einem Gemisch aus einem cyclischen Äther und einem aromatischen Kohlenwasserstoff wie Toluol, unter Verwendung eines Überschusses des Reduktionsmittels bei erhöhter Temperatur, beispielsweise bei Temperaturen zwischen 80 und 120 °C, durchgeführt.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Die Ausgangsverbindungen der Formel II sind bekannt und können nach an sich bekannten Verfahren hergestellt werden, beispielsweise durch Reduktion von Verbindungen der Formel V
(s. Formel V)
worin $R^1$, $R^2$ und $R^3$ obige Bedeutung besitzen. Verbindungen der Formel IV sind bekannt oder können auf an sich bekannte Weise erhalten werden, beispielsweise durch Umsetzung von Verbindungen der Formel V mit Verbindungen der Formel IIIb
(s. Formel IIIb)
worin $R^{4}$ " und X obige Bedeutung besitzen.

Die nachfolgenden Beispiele sollen die Erfindung näher erläutern, jedoch deren Umfang in keiner Weise beschränken.

Die folgenden Beispiele 1 bis 3 beschreiben erfindungsgemäße pharmazeutische Zubereitungen enthaltend einen Wirkstoff der Formel I sowie die Herstellung solcher pharmazeutischer Zubereitungen.

Beispiel 1:

| Tabletten | |
|---|---|
| Zusammensetzung: | |
| N,N'-Dicyclopropylmethyl-9,9-tetramethylen-3,7-diazabicyclo[ 3,3,1 ]nonan-dihydrochlorid | 20 Teile |
| Maisstärke | 30 Teile |
| Lactose | 55 Teile |
| Kollidon 25$^R$ | 5 Teile |
| Magnesiumstearat | 2 Teile |
| Talkum | 3 Teile |
| Gesamt | 115 Teile |

Herstellungsvorschrift:

Der Wirkstoff wird mit Maisstärke und feinpulvriger Lactose in einem Mischer vermischt. Die entstandene Mischung wird mit einer 20%-igen Lösung von Polyvinylpyrrolidon (Kollidon 25$^R$ der Fa. BASF) in entmineralisiertem Wasser durchfeuchtet. Falls erforderlich wird weiteres entmineralisiertes Wasser hinzugefügt. Das feuchte Granulat wird durch ein 2 mm-Sieb passiert, bei 40 °C auf Horden getrocknet und anschließend durch ein 1 mm-Sieb (Frewitt-Maschine) passiert. Nach dem Vermischen des Granulates mit Magnesiumstearat und Talkum werden daraus Tabletten mit einem Gewicht von 115 mg gepreßt, so daß jede Tablette 20 mg Wirkstoff enthält.

Beispiel 2:

| Kapseln | |
|---|---|
| Zusammensetzung: | |
| N-Isobutyl-N'-isopropyl-9,9-pentamethylen-3,7-diazabicyclo[ 3,3,1 ]nonan-dihydrogenfumarat | 20 Teile |
| Maisstärke | 20 Teile |
| Lactose | 45 Teile |
| Kollidon 25$^R$ | 3 Teile |
| Magnesiumstearat | 1,5 Teile |
| Aerosil 200$^R$ | 0.5 Teile |
| Gesamt | 90 Teile |

Herstellungsvorschrift:

Der Wirkstoff wird mit Maisstärke und feinpulvriger Lactose in einem Mischer vermischt. Die entstandene Mischung wird mit einer 20%-igen Lösung von Polyvinylpyrrolidon (Kollidon 25$^R$ der Fa. BASF) in entmineralisiertem Wasser durchfeuchtet. Falls erforderlich wird entmineralisiertes Wasser hinzugefügt. Das feuchte Granulat wird durch ein 1,6 mm-Sieb (Frewitt-Maschine) passiert, bei 40 °C auf Horden getrocknet und anschließend durch ein 1 mm-Sieb (Frewitt) passiert. Nach dem Vermischen des Granulates mit Magnesiumstearat und hochdisperser Kieselsäure (Aerosil 200$^R$, Fa. Degussa) füllt man davon jeweils 90 mg mittels automatischer Kapselmaschine in Hartgelatinekapseln der Größe 4 ab, so daß jede Kapsel 20 mg Wirkstoff enthält.

Beispiel 3:

| Ampullen | |
|---|---|
| Zusammensetzung (pro Ampulle): | |
| N,N'-Dicyclopropylmethyl-9,9-tetramethylen-3,7-diazabicyclo[ 3,3,1 ]nonan-dihydrochlorid | 5 mg |
| Natriumchlorid | 16 mg |
| Wasser für Injektionszwecke ad | 2,0 ml |

Herstellungsvorschrift:

Man löst Natriumchlorid in Wasser für Injektionszwecke auf, fügt den Wirkstoff hinzu und löst unter Rühren. Mit genügend Wasser für Injektionszwecke wird bis zum Endvolumen aufgefüllt. Der Ansatz wird durch ein Membranfilter 0,25 μ passiert. Man füllt in Braunglasampullen jeweils 2,15 ml ab und schmilzt sie zu. Bei 121 °C wird 30 min. lang mit Dampf sterilisiert. 2 ml Injektionslösung enthalten 5 mg Wirkstoff.

Die nachfolgenden Beispiele sollen die Herstellung der Verbindungen der Formel I näher erläutern.

Beispiel 4:

3-Butyl-7-(2,5-dimethylbenzyl)-9,9-dimethyl-3,7-diazabicyclo[ 3,3,1 ]nonan = Testsubstanz Nr. 19.

3,5 g 3-n-Butyl-9,9-dimethyl-3,7-diazabicyclo[ 3,3,1 ]nonan wurden in 25 ml Dimethylformamid gelöst, und die Lösung wurde mit 0,8 g Lithiumamid versetzt. Sodann wurde der Reaktionsansatz 1 Stunde auf einer Temperatur von 60 °C gehalten und anschließend abkühlen gelassen. Nach dem Abkühlen wurde tropfenweise eine Lösung von 5,6 g 2,5-Dimethylbenzylchlorid in 10 ml Dimethylformamid zugegeben und das Reaktionsgemisch weitere 4 Stunden bei 80 °C gerührt. Anschließend wurde das Reaktionsgemisch mit wäßriger Zitronensäurelösung versetzt, wobei die Titelverbindung als zitronensaures Salz in Lösung ging. Die Lösung wurde mit Diäthyläther gewaschen und anschließend durch Zusatz von verdünnter Natronlauge alkalisch gestellt, wobei die Titelverbindung wieder als Base freigesetzt wurde. Diese wurde mit Diäthyläther extrahiert. Nach Trocknen des Ätherextraktes über Magnesiumsulfat wurde die ätherische Lösung filtriert und der Äther abdestilliert. Das zurückbleibende Öl wurde durch Destillation im Kugelrohr bei 210 °C, 0,01 Torr gereinigt.

Die als Öl erhaltene Titelverbindung wurde in Äther gelöst. Zu der Lösung wurde eine Lösung einer äquimolaren Menge Salicylsäure unter Rühren und Eiskühlung zugetropft. Das ausfallende Salicylatsalz wurde abfiltriert und getrocknet. Es wurden 2,1 g Salicylat der Titelverbindung mit einem Schmelzpunkt von 153-155 °C erhalten.

Beispiel 5:

3-Butyl-7-(2,5-dimethylbenzyl)-9,9-dimethyl-3,7-diazabicyclo[ 3,3,1 ]nonan = Testsubstanz Nr. 19.

A) 2,6 g 3-Butyl-9,9-dimethyl-2,4,6,8-tetraoxo-3,7-diazabicyclo-[ 3,3,1 ]nonan wurden in 50 ml Dimethylformamid mit 1,7 g Kaliumcarbonat eine Stunde auf 120 °C erhitzt. Nach dem Abkühlen wurde zu dem Reaktionsgemisch eine Lösung von 1,8 g 2,5-Dimethylbenzylchlorid in 20 ml Dimethylformamid zugetropft, und das Reaktionsgemisch weitere 3 Stunden auf 120 °C erhitzt. Zur Aufarbeitung wurde anschließend das Dimethylformamid abdestilliert und der Rückstand in Dichlormethan aufgenommen und mit auf einen alkalischen pH-Wert eingestelltem Wasser gewaschen. Die Dichlormethanphase wurde abgetrennt, über Magnesiumsulfat getrocknet und eingeengt. Nach Umkristallisation des als Rückstand verbleibenden Rohproduktes aus Äther/Aceton wurden 1,2 g 3-Butyl-7-(2,5-dimethylbenzyl)-9,9-dimethyl-2,4,6,8-tetraoxo-3,7-diazabicyclo[ 3,3,1 ]nonan mit einem Schmelzpunkt von 159-161 °C erhalten.

B) 5 g Lithiumaluminiumhydrid in einer Mischung aus 140 ml absolutem Tetrahydrofuran und 60 ml absolutem Toluol wurden im Ölbad erhitzt (Ölbadtemperatur 80 °C). Bei dieser Ölbadtemperatur wurde langsam eine Lösung von 11,2 g 3-Butyl-7-(2,5-dimethylbenzyl)-9,9-dimethyl-2,4,6,8-tetraoxo-3,7-diazabicyclo[ 3,3,1 ]nonan in einer Mischung aus 70 ml Tetrahydrofuran und 30 ml Toluol zugetropft. Das Reaktionsgemisch wurde weitere 6 Stunden bei einer Temperatur von 120 °C gehalten. Zur Aufarbeitung wurde anschließend unter basischen Bedingungen hydrolysiert, indem dem Reaktionsgemisch nacheinander 5 ml Wasser, 5 ml 15 %-ige wäßrige Natronlauge und nochmals 15 ml Wasser zugesetzt wurden. Der gebildete Niederschlag wurde abfiltriert. Das Filtrat wurde mehrmals mit Dichlormethan extrahiert. Die vereinigten Dichlormethanextrakte wurden über Magnesiumsulfat getrocknet und eingeengt. Das zurückbleibende Öl wurde durch Destillation im Kugelrohr bei 210 °C, 0,01 Torr gereinigt. Die so erhaltene ölige Titelverbindung wurde wie in Beispiel 4 beschrieben mit Salicylsäure zu ihrem Monosalicylat umgesetzt. Ausbeute 6,8 g Salicylat der Titelverbindung mit einem Schmelzpunkt von 153-155 °C.

Nach den in den Beispielen 4 und 5 beschriebenen Methoden wurden auch die folgenden Verbindungen der Formel I erhalten. Die Testsubstanznummern beziehen sich auf die vorstehende Tabelle I.

| Test Subst. Nr. | $R^1$ | $R^2$ | $R^3$ | $R^4$ = $R^{5'}$-phen-$CH_2$- $R^{5'}$ = | Fp des Monosalicylat in °C |
|---|---|---|---|---|---|
| 16 | n-$C_4H_9$- | $CH_3$- | $CH_3$- | 3-Cl | 136-139 |
| 17 | n-$C_4H_9$- | $CH_3$- | $CH_3$- | 2-Cl | 146-147 |
| 18 | n-$C_4$ $H_9$- | $CH_3$- | $CH_3$- | 4-$CH_3$ O- | 91- 95 |
| 20 | n-$C_4$ $H_9$- | $CH_3$- | $CH_3$- | 4-$CH_3$- | 158-160 |

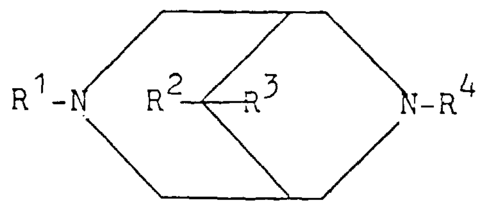

I

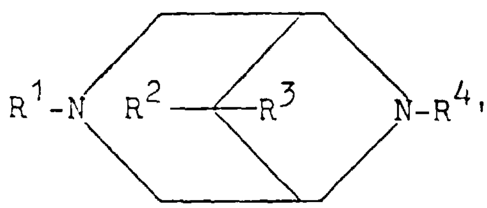

Ia

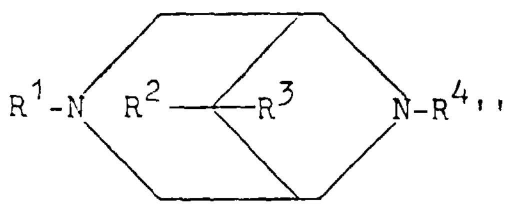

Ib

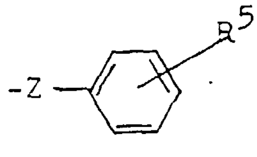

a

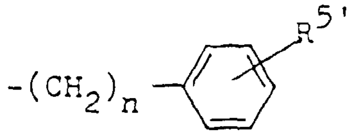

a'

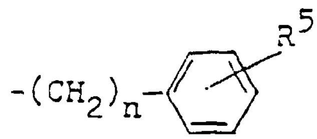

a''

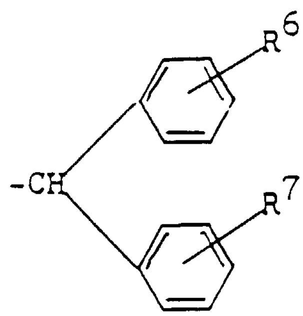

b

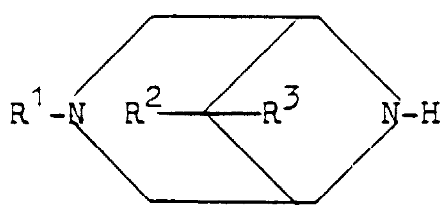

II

$R^4$-X III

$R^{4'}$-X IIIa

$R^{4''}$-X IIIb

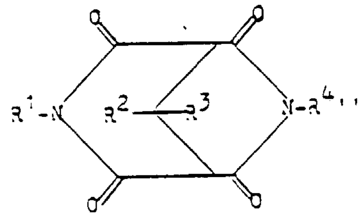

IV

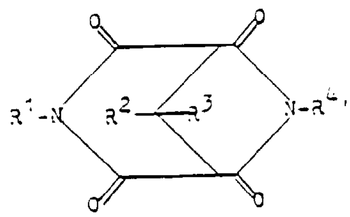

IVa

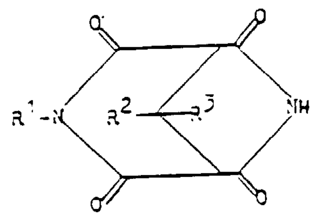

V

## Patentansprüche

1. Verwendung von 3,7-Diazabicyc1o[3,3,1]nonan-Verbindungen der allgemeinen Formel I

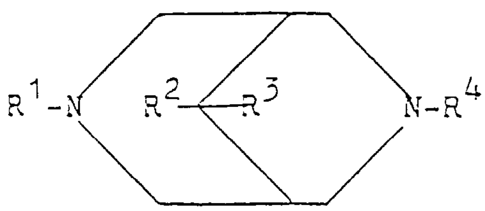

I

worin

| | |
|---|---|
| $R^1$ | eine Alkylgruppe mit 1 - 6 Kohlenstoffatomen, eine Alkenylgruppe mit 3 - 6 Kohlenstoffatomen, deren Doppelbindung nicht direkt an das Stickstoffatom gebunden ist, eine Cycloalkylalkylgruppe mit 4 - 9 Kohlenstoffatomen oder Benzyl bedeutet, |
| $R^2$ | Alkyl mit 1 - 4 Kohlenstoffatomen bedeutet und |
| $R^3$ | Alkyl mit 1 - 4 Kohlenstoffatomen bedeutet oder |
| $R^2$ und $R^3$ | gemeinsam eine Alkylengruppe mit 3 - 6 Kohlenstoffatomen bilden, und |
| $R^4$ | eine Alkylgruppe mit 1 - 6 Kohlenstoffatomen, eine Alkenylgruppe mit 3 - 6 Kohlenstoffatomen, deren Doppelbindung nicht direkt an das Stickstoffatom gebunden ist, oder eine Cycloalkylalkylgruppe mit 4 - 9 Kohlenstoffatomen bedeutet, oder |
| $R^4$ | eine Gruppe der allgemeinen Formel a |

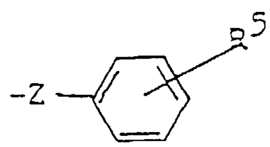

a

darstellt, worin

$R^5$ Wasserstoff, Halogen, Alkyl mit 1 - 4 Kohlenstoffatomen oder Alkoxy mit 1 - 4 Kohlenstoffatomen bedeutet und

Z eine Alkylenkette mit 1 - 3 Kohlenstoffatomen oder die Propenylenkette, deren Doppelbindung zum Phenylrest konjugiert ist, bedeutet, oder

$R^4$ eine Gruppe der allgemeinen Formel b

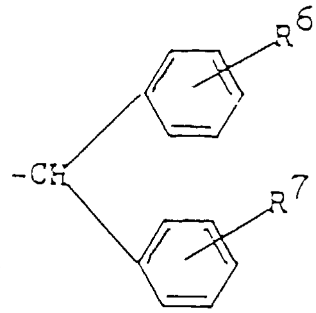

b

darstellt, worin

$R^6$ Wasserstoff, Halogen, Alkyl mit 1 - 4 Kohlenstoffatomen oder Alkoxy mit 1 - 4 Kohlenstoffatomen und

$R^7$ Wasserstoff, Halogen, Alkyl mit 1 - 4 Kohlenstoffatomen oder Alkoxy mit 1 - 4 Kohlenstoffatomen bedeuten,

und deren physiologisch verträglichen Säureadditionssalzen zur Herstellung von pharmazeutischen Zubereitungen zur diureseverstärkenden Behandlung von Krankheitszuständen, bei denen eine vermehrte Harnausscheidung und eine erhöhte Salzausscheidung zur Ausschwemmung von Ödemen erforderlich ist.

**2.** Verwendung von 3,7-Diazabicyclo[3,3,1]nonan-Verbindungen gemäß Anspruch 1, dadurch gekennzeichnet, daß Verbindungen verwendet werden, worin $R^2$, $R^3$ und $R^4$ die in Anspruch 1 angegebene Bedeutung besitzen und $R^1$ eine Alkylgruppe mit 1 - 6 Kohlenstoffatomen oder eine Cycloalkylalkylgruppe mit 4 - 7 Kohlenstoffatomen bedeutet.

**3.** Verwendung von 3,7-Diazabicyclo[3,3,1]nonan-Verbindungen gemäß Anspruch 2, dadurch gekennzeichnet, daß Verbindungen verwendet werden, worin $R^1$, $R^2$ und $R^3$ die in Anspruch 2 angegebene Bedeutung besitzen und $R^4$ Alkyl mit 1 - 6 Kohlenstoffatomen oder Cycloalkylalkyl mit 4 - 7 Kohlenstoffatomen oder eine Gruppe der Formel b bedeutet.

**4.** Verwendung von 3,7-Diazabicyclo[3,3,1]nonan-Verbindungen gemäß Anspruch 3, dadurch gekennzeichnet, daß Verbindungen verwendet werden, worin $R^2$ und $R^3$ die in Anspruch 3 angegebene Bedeutung besitzen, $R^1$ Alkyl mit 3 - 6 Kohlenstoffatomen oder Cycloalkylalkyl mit 4 - 7 Kohlenstoffatomen bedeutet und $R^4$ Alkyl mit 3 - 6 Kohlenstoffatomen oder Cycloalkylalkyl mit 4 - 7 Kohlenstoffatomen bedeutet.

**5.** Verwendung von 3,7-Diazabicyclo[3,3,1]nonan-Verbindungen gemäß Anspruch 4, dadurch gekennzeichnet, daß N,N'Dicyclopropylmethyl-9,9-tetramethylen-3,7-diazabicyclo-[3,3,1]nonan, N-Isobutyl-N'-isopropyl-9,9-pentamethylen-3,7-diazabicyclo[3,3,1]nonan und deren physiologisch verträgliche Säureadditionssalze verwendet werden.

**6.** 3,7-Diazabicyclo[3,3,1]nonan-Verbindungen der allgemeinen Formel Ia

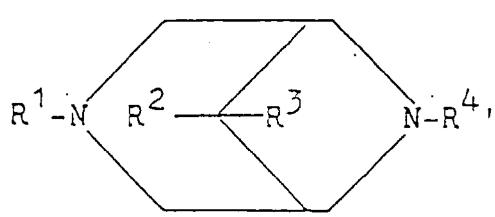

Ia

worin

$R^1$, $R^2$ und $R^3$ die in Anspruch 1 angegebene Bedeutung besitzen und

$R^{4'}$ eine Gruppe der allgemeinen Formel a'

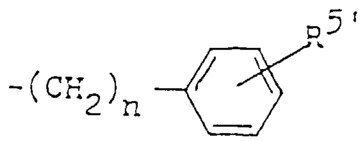

a'

darstellt, worin

n 1 - 3 bedeutet und
$R^{5'}$ Halogen, Alkyl mit 1 - 4 Kohlenstoffatomen, Alkoxy mit 1 - 4 Kohlenstoffatomen oder, falls n 2 oder 3 ist, auch Wasserstoff bedeutet,

und deren physiologisch verträgliche Säureadditionssalze.

**7.** Verfahren zur Herstellung der 3,7-Diazabicyclo[3,3,1]nonan-Verbindungen gemäß Anspruch 6 worin

$R^1$, $R^2$, $R^3$ und $R^{4'}$ die in Anspruch 6 angegebene Bedeutung besitzen

und deren physiologisch verträglichen Säureadditionssalzen, dadurch gekennzeichnet, daß man

a) Verbindungen der allgemeinen Formel II

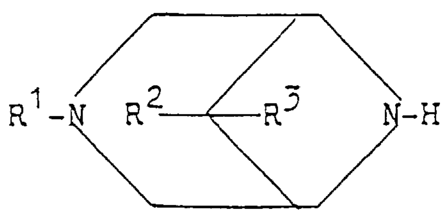

II

worin $R^1$, $R^2$ und $R^3$ obige Bedeutung besitzen, mit Verbindungen der allgemeinen Formel IIIa

$$R^{4'}\text{-X} \qquad \text{IIIa}$$

worin $R^{4'}$ obige Bedeutung besitzt und X eine aminolytisch abspaltbare Schutzgruppe bedeutet, umsetzt, oder

b) Verbindungen der allgemeinen Formel IVa

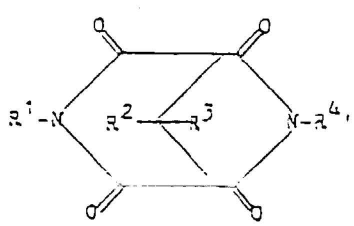

IVa

worin $R^1$, $R^2$, $R^3$ und $R^{4'}$ obige Bedeutung besitzen, reduziert und gewünschtenfalls freie Verbindungen der Formel Ia in ihre physiologisch verträglichen Säureadditionssalze überführt oder die Säureadditionssalze in die freien Verbindungen der Formel Ia überführt.

## Claims

1. Use of 3,7-diazabicyclo[3.3.1]nonane compounds of the general Formula I,

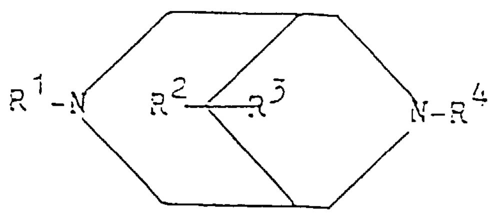

I

wherein

| | |
|---|---|
| $R^1$ | is an alkyl group with 1-6 carbon atoms, an alkenyl group with 3-6 carbon atoms, the double bond of which is not linked directly to the nitrogen atom, a cycloalkylalkyl group with 4-9 carbon atoms or benzyl, |
| $R^2$ | is alkyl with 1-4 carbon atoms and |
| $R^3$ | is alkyl with 1-4 carbon atoms or |
| $R^2$ and $R^3$ | together form an alkylene chain with 3-6 carbon atoms, and |
| $R^4$ | is an alkyl group with 1-6 carbon atoms, an alkenyl group with 3-6 carbon atoms, the double bond of which is not linked directly to the nitrogen atom, or a cycloalkylalkyl group with 4-9 carbon atoms, or |
| $R^4$ | is a group of the general Formula a, |

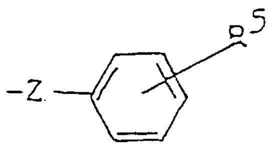

a

wherein

$R^5$ is hydrogen, halogen, alkyl with 1-4 carbon atoms or alkoxy with 1-4 carbon atoms and
Z is an alkylene chain with 1-3 carbon atoms or the propenylene chain, the double bond of which is conjugated to the phenyl radical, or

$R^4$ is a group of the general Formula b,

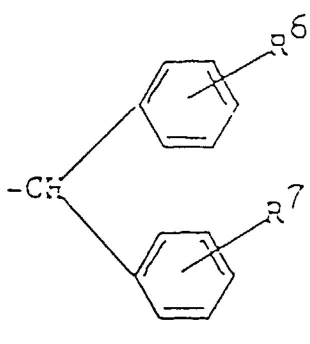

b

wherein

$R^6$ is hydrogen, halogen, alkyl with 1-4 carbon atoms or alkoxy with 1-4 carbon atoms and
$R^7$ is hydrogen, halogen, alkyl with 1-4 carbon atoms or alkoxy with 1-4 carbon atoms,

and their physiologically compatible acid addition salts for the production of pharmaceutical preparations for the

diuresis-increasing treatment of conditions in which increased elimination of urine and increased elimination of salts is required in order to reduce oedemas.

**2.** Use of 3,7-diazabicyclo[3.3.1]nonane compounds according to Claim 1, characterised in that compounds are used in which $R^2$, $R^3$ and $R^4$ have the meanings given in Claim 1 and $R^1$ is an alkyl group with 1-6 carbon atoms or a cycloalkylalkyl group with 4-7 carbon atoms.

**3.** Use of 3,7-diazabicyclo[3.3.1]nonane compounds according to Claim 2, characterised in that compounds are used in which $R^1$, $R^2$ and $R^3$ have the meanings given in Claim 2 and $R^4$ is alkyl with 1-6 carbon atoms or cycloalkylalkyl with 4-7 carbon atoms or a group of Formula b.

**4.** Use of 3,7-diazabicyclo[3.3.1]nonane compounds according to Claim 3, characterised in that compounds are used in which $R^2$ and $R^3$ have the meanings given in Claim 3, $R^1$ is alkyl with 3-6 carbon atoms or cycloalkylalkyl with 4-7 carbon atoms and $R^4$ is alkyl with 3-6 carbon atoms or cycloalkylalkyl with 4-7 carbon atoms.

**5.** Use of 3,7-diazabicyclo[3.3.1]nonane compounds according to Claim 4, characterised in that N,N'-dicyclopropylmethyl-9,9-tetramethylen-3,7-diazabicyclo[3,3,1]nonane, N-isobutyl-N'-isopropyl-9,9-pentamethylen-3,7-diazabicyclo[3.3.1]nonane and their physiologically compatible acid addition salts are used.

**6.** 3,7-diazabicyclo[3.3.1]nonane compounds of the general Formula Ia,

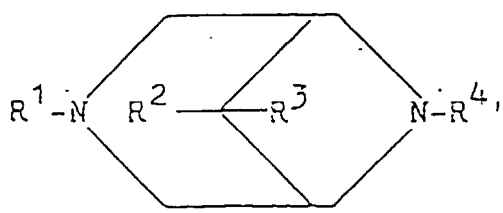

Ia

wherein
$R^1$, $R^2$ and $R^3$ have the meanings given in Claim 1, and
$R^{4'}$ is a group of the general Formula a',

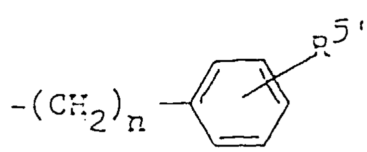

a'

wherein

n is 1-3 and
$R^{5'}$ is halogen, alkyl with 1-4 carbon atoms, alkoxy with 1-4 carbon atoms or, if n is 2 or 3, also hydrogen,

and their physiologically compatible acid addition salts.

**7.** Method for producing the 3,7-diazabicyclo[3.3.1]-nonane compounds according to Claim 6, wherein $R^1$, $R^2$, $R^3$ and $R^{4'}$ have the meanings given in Claim 6, and their physiologically compatible acid addition salts, characterised in that

a) compounds of the general Formula II,

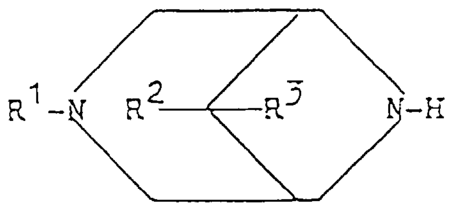

II

wherein $R^1$, $R^2$ and $R^3$ have the above meanings, are reacted with compounds of the general Formula IIIa,

$R^{4'}$-X  IIIa

wherein $R^{4'}$ has the above meaning and X is an aminolytically cleavable protective group, or

b) compounds of the general Formula IVa,

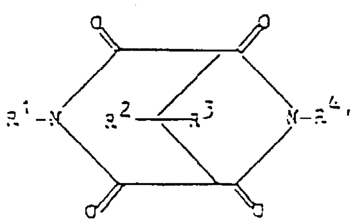

IVa

wherein $R^1$, $R^2$, $R^3$ and $R^{4'}$ have the above meanings,
are reduced,

and if desired free compounds of Formula Ia are converted into their physiologically compatible acid addition salts or the acid addition salts are converted into the free compounds of Formula Ia.

## Revendications

**1.** Utilisation de composés de 3,7- diazabicyclo [3,3,1]nonane de formule générale I

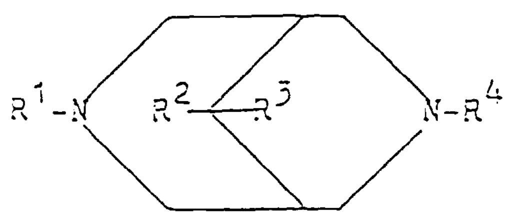

I

dans laquelle

| | |
|---|---|
| $R^1$ | est un groupe alkyle ayant de 1 à 6 atomes de carbone, un groupe alcényle ayant de 3 à 6 atomes de carbone, dont la double liaison n'est pas liée directement à l'atome d'azote, un groupe cycloalkylalkyle ayant de 4 à 9 atomes de carbone, ou un groupe benzyle, |
| $R^2$ | est un groupe alkyle en $C_1$-$C_4$ et |
| $R^3$ | est un groupe alkyle en $C_1$-$C_4$, ou bien |
| $R^2$ et $R^3$ | forment ensemble une chaîne alcylène ayant de 3 à 6 atomes de carbone, et |
| $R^4$ | représente un groupe alkyle ayant de 1 à 6 atomes de carbone, un groupe alcényle ayant de 3 à 6 atomes de carbone dont la double liaison n'est pas liée directement à l'atome d'azote ou un troupe |

cycloalkylalkyle ayant de 4 à 9 atomes de carbone, ou

$R^4$ représente un groupe de formule générale a

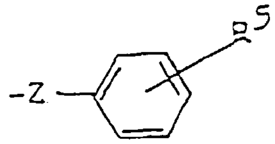

a

dans laquelle

$R^5$ représente un atome d'hydrogène ou d'halogène, un groupe alkyle avec 1 à 4 atomes de carbone ou alcoxy avec 1 à 4 atomes de carbone, et

Z représente une chaîne alcylène ayant de 1 à 3 atomes de carbone ou la chaîne propénylène, dont la double liaison est conjuguée au radical phényle, ou

$R^4$ représente un groupe de formule générale b

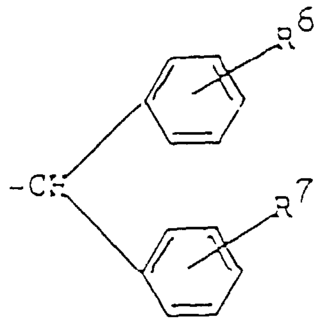

b

dans laquelle

$R^6$ représente un atome d'hydrogène, d'halogène, un groupe alkyle avec 1 à 4 atomes de carbone ou alcoxy avec 1 à 4 atomes de carbone, et

$R^7$ représente un atome d'hydrogène, d'halogène, un groupe alkyle avec 1 à 4 atomes de carbone ou un alcoxy avec 1 à 4 atomes de carbone,

et de leurs sels d'addition d'acide physiologiquement compatibles pour la production de préparations pharmaceutiques pour le traitement renforçant la diurèse des états pathologiques, dans lesquels une élimination augmentée de l'urine et une élimination augmentée de sels sont indispensables pour la suppression des oedèmes.

**2.** Utilisation de composés de 3,7 diazabicyclo [3,3,1]nonane selon la revendication 1, caractérisée en ce que l'on utilise des composés dans lesquels $R^2$, $R^3$ et $R^4$ ont les significations indiquées dans la revendication 1 et $R^1$ représente un groupe alkyle ayant de 1 à 6 atomes de carbone, ou un groupe cycloalkylalkyle ayant de 4 à 7 atomes de carbone.

**3.** Utilisation de composés de 3,7 diazabicyclo [3,3,1]nonane selon la revendication 2, caractérisée en ce que l'on utilise des composés dans lesquels $R^1$, $R^2$ et $R^3$ ont les significations indiquées à la revendication 2 et $R^4$ représente une groupe alkyle ayant de 1 à 6 atomes de carbone ou un cycloalkylalkyle ayant de 4 à 7 atomes de carbone ou un groupe de formule b.

**4.** Utilisation de composés de 3,7 diazabicyclo [3,3,1]nonane selon la revendication 3, caractérisée en ce que l'on utilise des composés dans lesquels $R^2$ et $R^3$ ont les significations indiquées dans la revendication 3, $R^1$ représente un groupe alkyle ayant de 3 à 6 atomes de carbone ou un groupe cycloalkylalkyle ayant de 4 à 7 atomes de carbone et $R^4$ représente un alkyle ayant de 3 à 6 atomes de carbone ou un cycloalkylalkyle ayant de 4 à 7 atomes de carbone.

**5.** Utilisation de composés de 3,7 diazabicyclo [3,3,1]nonane selon la revendication 4, caractérisée en ce que l'on

utilise le N, N'-dicyclopropylméthyl-9,9-tétraméthylène-3,7-diazabicyclo[3,3,1]nonane, le N-isobutyl-N'-isopropyl-9,9-pentaméthylène-3,7-diazabicyclo[3,3,1]nonane, et leurs sels d'addition d'acide physiologiquement compatibles.

**6.** Composés de 3,7-diazabicyclo[3,3,1]nonane de formule générale Ia

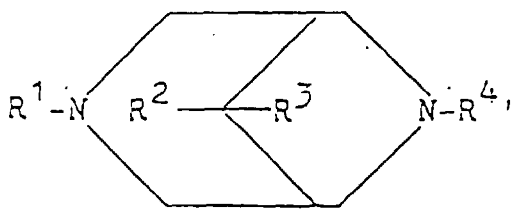

**Ia**

dans laquelle

$R^1$, $R^2$ et $R^3$ ont les significations indiquées dans la revendication 1, et
$R^{4'}$ représente une groupe de formule générale a'

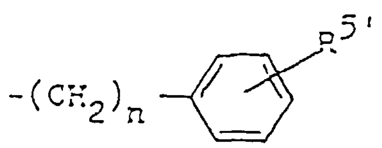

**a'**

dans laquelle,

n représente un nombre de 1 à 3 et
$R^{5'}$ représente un atome d'halogène, un groupe alkyle avec 1 à 4 atomes de carbone, un groupe alcoxy avec 1 à 4 atomes de carbone ou, dans le cas où n est égal à 2 ou 3, également de l'hydrogène,

et leurs sels d'addition d'acide physiologiquement compatibles.

**7.** Procédé de préparation des composés de 3,7-diazabicyclo[3,3,1]nonane selon la revendication 6,

dans lesquels $R^1$, $R^2$, $R^3$ et $R^4$ ont les significations indiquées dans la revendication 6
et de leurs sels d'addition d'acide physiologiquement compatibles, caractérisé en ce que :

a) on fait réagir des composés de formule générale II

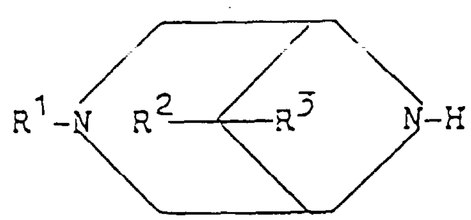

**II**

où $R^1$, $R^2$ et $R^3$ ont les significations précitées, avec des composés de formule générale IIIa

$$R^{4'}\text{-}X; \qquad \text{IIIa}$$

où $R^{4'}$ a la signification précitée et X représente un groupe de protection séparable par aminolyse, ou

b) on réduit des composés de formule générale IVa

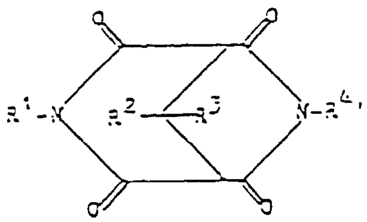

IVa

où $R^1$, $R^2$, $R^3$ et $R^{4'}$ ont les significations précitées et on transforme éventuellement les composés libres de formule Ia en leurs sels d'addition d'acide physiologiquement compatibles ou les sels d'addition d'acide en composés libres de formule Ia.